# EUROPEAN PATENT APPLICATION

(11) **EP 1 600 063 A1**
(43) Date of publication of application: **30.11.2005**
(21) Application number: 05003739.9
(22) Date of filing: 22.02.2005
(51) Int. Cl.: A23L 1/30, A61K 31/22, A61P 3/00, A61P 43/00

(54) **Composition to improve the endurance**

(30) Priority: 24.02.2004 JP 2004047348
(71) Applicant: KAO CORPORATION, Tokyo 103-8210 (JP)
(72) Inventor: Murase, Takatoshi, Sumida-ku Tokyo 131-8501 (JP); Harada, Ushio, Sumida-ku Tokyo 131-8501 (JP); Osaki, Noriko, Sumida-ku Tokyo 131-8501 (JP); Ichiro, Tokimitsu, Sumida-ku Tokyo 131-8501 (JP)
(74) Representative: HOFFMANN EITLE

(57) **Abstract**

There are provided an endurance improver and an anti-fatigue agent for broadly-defined exercises including sports requiring endurance and labors requiring repeated muscle exertions. The present endurance improver and anti-fatigue agent contain diacylglycerol as an active ingredient.

## Description

### Field of the Invention

The present invention relates to an endurance improver or an anti-fatigue agent oriented toward physical exercises including sports and labors in a broad sense.

### Background of the Invention

For those people involved in broadly-defined exercises including sports requiring endurance and labors requiring repeated muscle exertions, it is strongly desired to improve their physical endurance in such sports or labors to inhibit or deter their physical fatigues.

In this view, a wide variety of plants and other materials have been searched for any ingredients or substances having actions of improving physical endurance or inhibiting physical fatigues. For example, Varnished Conk (Reishi) ingredients (cf. Patent literature 1), Crataegus extracts (cf. Patent literature 2) and so on have so far been reported as having actions of improving physical endurance. Further, substances that have been reported as having actions of anti-fatigue agents include biotin (Patent literature 3), certain amino acid compositions (Patent literature 4), and 2-ketoglutaric acid (Patent literature 5).

Meanwhile, diacylglycerol that is contained in a small quantity in vegetable oils is reported to have a depressant action on postprandial increase in blood neutral fat and an action to reduce body fat in animals or humans (cf. nonpatent literatures 1 through 3). However, how the diacylglycerol influences on the physical endurance or fatigues during exercises remains unknown at all.
[Patent literature 1] Japanese published unexamined patent application 05-123135
[Patent literature 2] Japanese published unexamined patent application 08-47381
[Patent literature 3] Japanese published unexamined patent application 06-305963
[Patent literature 4] Japanese published unexamined patent application 07-25838
[Patent literature 5] Japanese published unexamined patent application 10-175855
[Nonpatent literature 1] Inform, Vol. 12, 1098-1102, 2001
[Nonpatent literature 2] Current Opinion in Lipidology, Vol. 14, 29-33, 2003
[Nonpatent literature 3] Monthly BIOINDUSTRY, Vol. 17(3), 52-59, 2000, by CMC Publishing Co., Ltd., Tokyo, Japan

### SUMMARY OF THE INVENTION

The present invention provides an endurance improver and an anti-fatigue agent oriented toward exercises which contain diacylglycerol as an active ingredient thereof.

The present invention also provides use of diacylglycerol for the manufacture of endurance improvers and anti-fatigue agents.

Further, the present invention provides a method of improving physical endurance and fatigues by administering an effective dose of diacylglycerol.

### DETAILED DESCRIPTION OF THE INVENTION

The inventor have studied diacylglycerol for its physiological actions and unexpectedly found out that diacylglycerol has excellent actions of improving physical endurance and inhibiting or retarding physical fatigues.

According to the present invention, there are provided medicines, foods and beverages that have actions of improving physical endurance and/or inhibiting physical fatigues for those people involved in broadly-defined exercises including sports requiring endurance and labors requiring repeated muscle exertions.

Fatty acid(s) constituting the diacylglycerol used for the endurance improver or anti-fatigue agent according to the present invention has a number of carbon atoms preferably ranging from 8 to 24 and more preferably from 16 to 22. The diacylglycerol contains unsaturated fatty acid(s) preferably in the range of 70 to 100 % by weight (hereinafter to be indicated simply as wt%) of the total content of their constituent fatty acids, more preferably from 80 to 100 wt% and even more preferably from 93 to 100 wt%. Further, for the present invention, the diacylglycerol has a (cis-unsaturated fatty acids content) vs. (trans-unsaturated fatty acids content + saturated fatty acids content) ratio of 5.5 or above. More preferably this ratio ranges from 8 to 25 and furthermore preferably from 9 to 20. Besides, it is preferred that the trans-unsaturated fatty acids content of the diacylglycerol be up to 5 wt% and that the saturated fatty acids content thereof be up to 5 wt% as well. Diacylglycerol exists as either 1,3-diacylglycerol or 1,2-diacylglycerol (2,3-diacylglycerol). From the viewpoint of efficacy, the diacylglycerol has preferably a 1,3-diacylglycerol content of at least 50 wt%, more preferably at least 55 wt% and even more preferably at least 60 wt%.

The diacylglycerol used for the present invention may be produced, for example, by interesterifying any fats and oils containing intended constituent fatty acid(s) with glycerin or by subjecting a mixture of intended constituent fatty acid(s) or ester(s) thereof and glycerin to esterification under the action of lipase. The latter esterification method employing lipase is preferable in view of preventing any isomerization from occurring during reaction. Even for the esterification method using lipase, it is also preferred, for the prevention of any isomerization in a refining means after isomerization process, that the refining means is operated under such moderate conditions as to prevent such isomerization from occurring.

Thus, it is preferred to use the diacylglycerol as a composition of fats and oils (also referred to herein as fats and oils compositions) containing also triacylglycerol or like ingredients. In view of efficacy, such a composition of fats and oils preferably contains from 5 to 100 wt% of diacylglycerol, more preferably from 15 to 99 wt%, further preferably from 40 to 95 wt% and even more preferably from 60 to 95 wt% of diacylglycerol.

While such a composition of fats and oils may contain triacylglycerol, the fats and oils preferably contain from 0 to 95 wt%, more preferably from 1 to 85 wt% and even more preferably from 5 to 60 wt% of triacylglycerol, in view of efficacy, flavor and oxidation stability of the resultant products. Considering efficacy, flavor and eating texture, constituent fatty acid(s) of the triacylglycerol contains unsaturated fatty acid(s) having a number of carbon atoms ranging from 16 to 22 preferably in a quantity ranging from 55 to 100 wt%, more preferably from 70 to 100 wt%, further preferably from 80 to 100 wt% and even more preferably from 90 to 97 wt%.

Further, the compositions of fats and oils may contain monoacylglycerol and in view of flavor and oxidation stability of the resultant products its content preferably ranges from 0 to 30 wt%, more preferably from 0.1 to 10 wt%, further preferably from 0.1 to 5 wt%, furthermore preferably from 0.1 to 2 wt% and even more preferably from 0.1 to 1.5 wt%. It is also preferred for convenience of manufacture that the monoacylglycerol has the same constituent fatty acid(s) as the diacylglycerol.

Since the above-described composition of fats and oils contains free fatty acids having a foreign taste, the free fatty acids content is limited preferably to 10 wt% or below, more preferably to 5 wt% or below, further preferably to 2.5 wt% or below, furthermore preferably to 1 wt% or below and even more preferably to about 0.5 wt% or below.

Further according to the present invention, it is preferable to add one or more antioxidants to the above composition of fats and oils in order to improve oxidation stability. Such preferable antioxidants include butylated hydroxyanisole (BHA), butylated hydroxytoluene (BHT), vitamin A, vitamin C, vitamin E, phospholipids, polyphenols and the like, including any combinations of two or more of them.

It is also preferable to further add one or more crystallization inhibitors to the above composition of fats and oils. Crystallization inhibitors preferably usable for the present invention include polyol fatty acid esters such as polyglycerin-condensed ricinoleic acid esters, polyglycerin fatty acid esters, sucrose fatty acid esters, sorbitan fatty acid esters, polyoxyethylene sorbitan fatty acid esters, propylene glycol fatty acid esters or the like. The crystallization inhibitors may be used in combination of two or more of them. The crystallization inhibitors content of the fats and oils composition ranges preferably from 0.02 to 0.5 wt% and more preferably from 0.05 to 0.2 wt%.

Generally, raw vegetable oils contain about 0.05 to about 1.2 wt% of phytosterols. However, the phytosterols contents of specific fats and oils compositions vary with specific processes by which they are produced. For example, if any commercially available distilled fatty acid is used as a raw material, the resultant composition of fats and oils will have a reduced phytosterols content. In this regard, it is preferable to use a fats and oils composition containing a phytosterol in a quantity of 0.05 wt% or above and more preferably 0.3 wt% or above. As the phytosterol content, the range of 0.05 to 20 wt% is satisfactory, although its upper limit is not specifically limited. Preferable phytosterols herein include unesterified free compounds such as, for example, α-sitosterol, β-sitosterol, stigmasterol or campesterol, α-sitostanol, β-sitostanol, stigmastanol, campestanol or cycloartenol, and esterified compounds including their fatty acid esters, ferulic acid esters, cinnamic acid esters or like esters.

For administering the endurance improver or anti-fatigue agent to humans, it is administered typically preferably in a quantity of 0.1 to 25 g/day and more preferably 1 to 15 g/day as diacylglycerol content per adult once a day or by dividing the daily dosage a few times. In order for it to exhibit its effective action more clearly, it is preferable to administer the present endurance improver or anti-fatigue agent 1 g/day or more as diacylglycerol.

The endurance improver or anti-fatigue agent of the present invention may be administered in the form of orally administered agents or drugs including for example solid preparations such as powders, granules, capsules, pills or tablets, and liquid formulations such as poti, suspensions or emulsions. When producing such orally administered agents, any commonly used additives may be mixed therewith depending on the type of such specific agents to be produced, including excipients, disintegrating agents, binders, lubricants, surfactants, alcohols, water, water-soluble polymers, sweeteners, flavoring agents, acidifiers and the like.

The endurance improver or anti-fatigue agent of the present invention may be taken in as foods or beverages. For giving as foods, any processed foods of fats and oils containing the present endurance improver or anti-fatigue agent may be used, including for example such health foods, functional foods and foods for specified health uses that exhibit specified functions to achieve health promotion. More specifically, such foods include, for example: capsules, tablets, granules; bakery foods such as breads or cakes, cookies, pies and bakery mix products; dressings such as French dressings; oil-in-water emulsion foods such as mayonnaises; water-in-oil emulsion foods such as margarines and spreads; confectioneries such as creams, chocolates, potato chips, ice creams, desserts, and; food ingredients or materials such as sauces, coffee whiteners, whipped creams, grillade sauces, peanut butters, frying shortenings, baking shortenings, processed meat products, frozen foods, and cooking oils used for tempuras (Japanese deep-fried foods), fries, stir-fried foods or the like. Such foods may be produced by adding any commonly used food ingredients depending on specific food types in addition to the fats and oils composition as described previously. The content of the present endurance improver or anti-fatigue agent in foods or beverages ranges typically preferably from 0.05 wt% to 100 wt% and more preferably from 0.5 wt% to 80 wt%, although it may vary depending on specific food types.

### Examples

### EXAMPLE 1 (endurance improving and fatigue inhibiting actions of diacylglycerol on rats):

As a material containing diacylglycerol, Econa Cooking Oil produced by Kao Corporation (Tokyo, Japan) was used. As its control, triacylglycerol composed of substantially the same fatty acids as the diacylglycerol was used.

Rats (SD rats, male, 4 weeks old) were reared for 4 weeks on a diacylglycerol food (DG food) or on a triacylglycerol food (TG food) as shown in Table 1 below, respectively. After 2 weeks of rearing, the rats were subjected to a treadmill running test over the succeeding third and fourth weeks (on Treadmill KN-73 made by NATSUME-Seisakusho, Tokyo, Japan; 5 times a week for 10 minutes each time at belt speed of 20 m/min.). Four weeks after the start of experiment, the limit running time was measured under the following conditions.

### Evaluation of limit running time (at 15 % belt inclination):

40 min. running at 22 m/min. ⇒ 5 min. running at 27 m/min. ⇒ 5 min. running at 22 m/min. ⇒ repetition of this running cycle
The time when a rat had come to be incapable of running any further was regarded as the point of its exhaustion, and the time elapsed until that point was used as the limit running time of the tested rat based on which the endurance improving and anti-fatigue actions were to be evaluated. The limit running time measured on and averaged for all rats tested are shown in Table 2 below.

**Table 1**

| (wt%) | | |
|---|---|---|
| | TG food | DG food |
| Casein | 20 | 20 |
| DL-methionine | 0.2 | 0.2 |
| Triacylglycerol | 10 | 0 |
| Diacylglycerol | 0 | 10 |
| Minerals | 4 | 4 |
| Vitamins | 2.2 | 2.2 |
| Cellulose powder | 8.1 | 8.1 |
| Potato starch | 55.5 | 55.5 |
| Total | 100 | 100 |

**Table 2**

| | Limit running time (min.) |
|---|---|
| TG food | 92.5 ± 25.4 |
| DG food | 126.9 ± 20.1 |

As clearly understood from the test result given in Table 2, the rats reared on the diacylglycerol food have a limit running time after four weeks of rearing that is significantly longer (p<0.01) than the rats reared on the control food (triacylglycerol food), thus showing that the diacylglycerol has significant actions of improving physical endurance and inhibiting physical fatigues.

## Claims

1. An endurance improver comprising diacylglycerol as an active ingredient.

2. An anti-fatigue agent comprising diacylglycerol as an active ingredient.

3. Use of diacylglycerol for the manufacture of an endurance improver.

4. The use according to claim 3, wherein the endurance improver is provided in the form of food or beverage.

5. Use of diacylglycerol for the manufacture of an anti-fatigue agent.

6. The use according to claim5, wherein the angi-fatigue agent is provide in the form of food or beverage.
